# EUROPEAN PATENT SPECIFICATION

(11) **EP 1 867 322 B1**
(45) Date of publication and mention of the grant of the patent: **20.01.2010**
(21) Application number: 07380166.4
(22) Date of filing: 11.06.2007
(51) Int. Cl.: A61K 9/06, A61K 31/17, A61K 31/573, A61K 31/60, A61P 17/06

(54) **Topical composition for the treatment of psoriasis**
Topische Zusammensetzung zur Behandlung von Schuppenflechte
Composition topique pour le traitement du psoriasis

(30) Priority: 12.06.2006 ES 200601574
(43) Date of publication of application: 19.12.2007
(73) Proprietor: Fernández Rodríguez, M. Cristina, 36320 Chapela, Pontevedra (ES); Barja Galvan, Emilio, 36320 Chapela, Pontevedra (ES)
(72) Inventor: Fernández Rodríguez, M. Cristina, 36320 Chapela, Pontevedra (ES); Barja Galvan, Emilio, 36320 Chapela, Pontevedra (ES)
(74) Representative: Rodriguez Perez, Jesus

(56) References cited:
- WO-A-96/01633
- WO-A-2006/042059
- WO-A-2006/102004
- ES-A1- 2 083 333
- ES-A1- 2 231 007
- US-A- 4 740 372
- FLUHR, JOACHIM W. ET AL: "Effects of moisturizer in psoriasis" 9 November 2005 (2005-11-09), DRY SKIN AND MOISTURIZERS (2ND EDITION) , 135-143. EDITOR(S): LODEN, MARIE; MAIBACH, HOWARD I. PUBLISHER: CRC PRESS LLC, BOCA RATON, FLA , XP009089242 ISBN: 0-8493-2134-4 * page 137, lines 3-6, paragraph 1 * * page 137, line 4, paragraph 6 *
- DATABASE EPODOC [Online] EUROPEAN PATENT OFFICE, THE HAGUE, NL; XP002449839 & BG 106 703 A (SOFARMA AD [BG]) 28 November 2003 (2003-11-28)
- MASON J ET AL: "Topical preparations for the treatment of psoriasis: a systematic review" BRITISH JOURNAL OF DERMATOLOGY, vol. 146, no. 3, March 2002 (2002-03), pages 351-364, XP002244836 ISSN: 0007-0963

## Description

The present invention relates to a new pharmaceutical composition for topical use for the symptomatic treatment of psoriasis.

Psoriasis, a chronic non-contagious inflammatory skin condition, is one of the most common skin disorders, representing one in five dermatological consultations with a universal distribution that affects 1-2% of the population, although it manifests itself with racial and geographical variations. It can affect any age group; some cases of psoriasis have been described in newborn infants and others have been diagnosed in people over the age of 100. The most common age is around 30, generally within the 20 to 50 age group.

Classically, psoriasis has been described as a disorder characterised by the existence of an epidermal hyperplasia resulting from alterations in cellular kinetics and from a differentiation that gives rise to hyperproliferation and hyperplasia. Various studies and observations have shown the involvement of T lymphocytes in the development of psoriasis lesions. Due to this and other information psoriasis is currently studied as a condition involving genetic, environmental and particularly immune factors. The disorder often develops in several members of the same family. Many genes in different chromosomes have been identified that contribute to the development of a psoriatic phenotype. For many years psoriasis was seen as a predominantly epidermal condition; however, different observations including the fact that psoriasis responds to various immunosuppressant treatments (cyclosporin, IL-2, Anti-CD4) or the fact that psoriasis disappears after a bone transplant from a healthy donor and appears after a transplant from a psoriasis-affected donor have changed this view, proving the role of T lymphocyte activation in the development of this condition.

There are several factors that trigger outbreaks of psoriasis, including physical traumas, bacterial infections (due to the production of superantigens), viral infections, situations of stress and potentially medications. The mechanism of action of some of these triggering factors is to initiate the immune process.

This condition is characterised by the appearance of red scaly plaques, mainly on the shoulders, knees, scalp and nails. It can also affect the joints (psoriatic arthritis). The most significant medical complications that accompany psoriasis are psoriatic arthropathy, erythrodermia, relapses and irritation.

The size of the lesions caused by this disorder can vary considerably, from small papules to large plaques covering large areas of the body, but they always present a similar clinical morphology. Patients with psoriasis can suffer from various different forms of the condition -plaque, guttate, erythrodermic and pustular psoriasis- that represent different manifestations of the same disorder. The extension of psoriasis is calculated using the PASI index (Psoriasis Area Severity Index), which considers the size, erythema, thickness and scales present in the affected area. The maximum index value is 72 and psoriasis is classed as mild (<10), moderate (10-50) and severe (>50), according to the index value.

Generally, psoriasis has few clinical symptoms and there is not usually much itching, except when it affects the palms of the hands, soles of the feet or folds of the skin, where it tends to cause localised discomfort and itching, which can occasionally be very intense. According to data provided by "Acción Psoriasis", up to 75% of those affected state that psoriasis is a significant problem in their lives, as the patient can feel socially isolated due to aesthetic and social rejection and rejection in the workplace, and it can even affect the person's ability to do his or her job, sex life, etc. In short, psoriasis reduces the patient's quality of life.

There are several ways of treating it that can make the lesions disappear or bring about a great improvement, including topical and systemic treatments and so-called "biological therapy", which is currently being developed. In the case of the latter treatment, it acts on the mechanisms that cause the symptoms of the disorder; however, its long-term efficacy and safety cannot be ensured and it is currently a very expensive alternative.

Although there is not yet a cure for psoriasis to speak of, the objective of current treatments is to free the skin of lesions for the longest possible period of time, which is called "clearing" the lesions or achieving their remission. The first step in treating psoriasis aims to clear the skin and the second step aims to keep it clear.

As regards topical treatment, it includes the application of topical corticosteroids, topical anthralin, coal tar and vitamin D derivatives. Topical corticosteroids have a multiple mechanism of action, with anti-inflammatory, antimitotic, immunomodulating and vasoconstrictive activity. As a general rule, it is better to use a milder steroid that is capable of achieving its purpose. Very strong halogenated steroids must only be used very occasionally, 50 g of clobetasol propionate, 0.05%, can suppress the patient's adrenal glands after a week.

Vitamin D derivatives improve the scales and the thickness of the plaques, but they are not very effective in improving the redness and, although they are helpful as a maintenance treatment for the condition and do not have the side effects that steroids have, their effectiveness alone is very limited and they tend to be administered in combination with topical corticosteroids and phototherapy, which means that these treatments are not very cost effective. The efficacy of phototherapy is based on the observation of the beneficial effect of sunlight (heliotherapy) on psoriasis patients. There are three methods of treatment: the Goeckerman method, which combines the application of coal tar and Ultraviolet B, the ingram method, which combines anthralin and UVB, and probably the easiest is PUVA therapy or photochemotherapy, which combines the administration of Psoralens and Ultraviolet A, thereby achieving an improvement of more than 90% in psoriasis patients. PUVA therapy has side effects during the treatment, such as nausea or a burning feeling on the skin and it also has long-term side effects, increasing the risk of developing squamous cell carcinomas, especially in the male genital area. In general, treatments with UV radiation can cause burns, ageing of the skin and immunosuppression, with a clear risk of developing skin carcinomas.

Coal tar is mainly applied in cases of psoriasis on the scalp. It is currently not very popular due to its unpleasant smell, because it stains skin and clothes and it can cause irritation.

Coal tar is used as a highly-concentrated short contact treatment, which is currently not very popular as it irritates the skin and stains cloths and objects.

Numerous topical compositions are disclosed in the state of the art for the treatment of psoriasis.

ES 2 231 007 (A1) discloses a topical cream comprising a combination of urea and salicylic acid. US-A-4 740 372 discloses a pharmaceutical composition for a topical administration comprising salicylic acid and betamethasone dipropionate. ES 2 083 333 (A1) discloses a cream for topical use comprising urea and betamethasone dipropionate.

Document US 2004/0225140 A1, for example, discloses a pharmaceutical composition for topical administration that comprises an effective amount of 17-clobetasol propionate and an effective amount of a hydroxy derivative of progesterone, the use of clobetasol propionate, a highly potent topical corticosteroid (European classification according to Miller and Munro, "Boletin de información Farmacoterapéutica de Navarra", vol. 9, no. 2 ), increases the risk of possible side effects deriving from the use of said corticosteroid. RO 120529 B1 discloses an antioxidant composition for topical use made from plant extracts whose scope of action is limited to regeneration (limitation of radical reactions) of the skin; whilst US 2006/0041016 A1, for example, claims a composition for topical administration containing prostaglandins, referring only to the treatment of inflammation but not to regeneration of the treated skin or to clearing its scaly appearance. In view of the above, there is a need for a composition for topical administration that covers all the symptomatic aspects of the disorder without having the disadvantages of the prior art.

One object of the present invention is therefore the formulation of a new composition for topical administration for the treatment of psoriasis that not only has therapeutic effects as an anti-inflammatory, antimitotic, immunomodulating and vasoconstrictive activity, but is also effective in terms of the visual and social aspect of the disorder, improving its symptoms and increasing the quality of life of the patient who is affected by it.

Given that with topical therapy the choice of excipient or vehicle is as important as the active substance, since it affects both its stability and its penetration, as well as providing the preparation with a different form of presentation (ointment, cream, gel, lotion, aerosol) and modifying its activity, another object of the invention is the formulation of a new composition for topical administration for the treatment of psoriasis wherein the vehicle and the excipients used therein, as well as the active substances, have a synergic activity that results in an optimum efficacy of the treatment in as short a period of time as possible.

The composition for topical administration of the present invention combines the advantageous properties of the vehicles and active substances, thus providing an effective composition for the treatment of psoriasis and minimising the risk of potential side effects associated with conventional treatments applied individually in the prior art.

The composition for topical administration for the treatment of psoriasis according to the present invention contains the following ingredients in the advantageous proportions specified below:
- Urea (between 3 and 9%)
- Salicylic acid (between 1 and 9%)
- Betamethasone dipropionate (between 0.1 and 0.9%)
- Propylene glycol (between 1 and 9%)
- Span-80 (between 3 and 12%)
- White petroleum jelly (between 6 and 19%)
- Isopropyl myristate (between 2 and 10%)
- Musk rose oil (approx. 2 to 3 drops)
- Water (q.s.)

The new composition of the invention combines the keratolytic, anti-inflammatory, antiseptic, emollient, moisturising and regenerative effects of all its ingredients, altogether increasing its beneficial effects on psoriasis.

The urea and salicylic acid contained in the composition of the invention, preferably in a proportion of between 3 and 9% and between 1 and 9%, respectively, are both agents with a keratolytic activity, favouring the removal of the scales and facilitating desquamation by dissolving the intracellular cement that joins the scales on the stratum corneum so that the corneocytes slough off and facilitating penetration of the active substances into the skin, as well as having moisturising and anti-inflammatory and antiseptic properties in the case of the salicylic acid.

The betamethasone dipropionate contained in the composition of the invention, preferably in a proportion of between 0.1 and 0.9%, is a topical corticosteroid with an anti-inflammatory, antipruritic, vasoconstrictive and immunosuppressant effect that is metabolised by the skin, for which no interaction with any other medication has yet been described. Its activity is increased by the occlusive effect of the isopropyl myristate, which is present in the composition of the invention in a preferable proportion of between 2 and 10%, as it increases its penetration into the stratum corneum. Furthermore, isopropyl myristate is an emollient that prevents water loss and favours the capacity to form or maintain a protective layer on the vehicle-skin interphase, which improves its moisturising properties (demulcent).

The addition of propylene glycol, preferably in a proportion of between 1 and 9%, as well as its characteristics as a normal vehicle in this type of compositions, helps to dry exudative dermatitis and reinforce the antiseptic and anti-inflammatory nature of the composition of the invention.

The white petroleum jelly is a normal ingredient in this type of topical W/O formulation and span-80 (sorbitan oleate), which is also normally used in this type of topical W/O formulation, is a non-ionic lipophilic surfactant that acts as an emulsifier and increases the water absorption capacity of excipients such as petroleum jelly.

The musk rose oil added to the composition of the invention enhances its properties due to its high content of essential polyunsaturated fatty acids (mainly linoleic and linolenic acids), which are beneficial for regenerating the skin and which energise the fibroblasts and the cells that produce collagen and elastin, and it also provides natural tocopherols, carotenes and tretinoin (transretinoic acid), improving the appearance and smoothness of the skin. It also gives the composition of the invention its characteristic gentle smell.

In an example of a preferred embodiment of the composition of the invention it is formulated as a cream or ointment.

All the percentages cited herein are weight percentages unless stated otherwise.

### Example of an embodiment of the composition of the invention in cream form

Crystalline urea is poured into a porcelain mortar and crushed to obtain a very fine powder. Then warm water is added until it has completely dissolved. The propylene glycol is added next, stirring at the same time in order to achieve a homogenous solution.

The white petroleum jelly is melted in a beaker over a bath of hot water, keeping the temperature at between 40 deg. C and 50 deg. C. When it is fluid, the warm bath is removed and the Span-80 is added to the beaker, stirring at the same time to achieve a homogenous cream. While it is still warm, the isopropyl myristate is added, stirring gently. The cream thus produced is left to stand at room temperature for approximately 10 min.

The oil phase (white petroleum jelly, span-80, isopropyl myristate) is now slowly poured into the mortar containing the aqueous phase (urea, water, propylene glycol), stirring until it forms a cream with a light homogenous consistency.

The right amounts of betamethasone dipropionate and salicylic acid are mixed thoroughly in a watch glass, until a homogenous mixture is achieved. This mixture is then added to the cream produced earlier. Small volumes of water are gradually and slowly added to the mixture to aid the addition of ingredients until the desired consistency is achieved. Finally, the musk rose oil (between 0.01 and 5.00 ml) is added to the final mixture to achieve the topical composition for the treatment of psoriasis of the invention.

## Claims

1. Pharmaceutical composition for topical use in the symptomatic treatment of psoriasis, **characterised in that** it contains the following ingredients in the specified proportions: urea (between 3 and 9% w/w), salicylic acid (between 1 and 9% w/w), betamethasone dipropionate (between 0.1 and 0.9% w/w), propylene glycol (between 1 and 9% w/w), Span-80 (between 3 and 12% w/w), white petroleum jelly (between 6 and 19% w/w), isopropyl myristate (between 2 and 10% w/w), musk rose oil (approx. 2 to 3 drops) and water.

2. Pharmaceutical composition for topical use in the treatment of psoriasis according to claim 1, **characterised in that** it is in cream form.

## Patentansprüche

1. Pharmazeutische Zusammensetzung für die topische Anwendung zur symptomatischen Behandlung der Psoriasis, **dadurch gekennzeichnet, dass** sie die folgenden Inhaltsstoffe in den angegebenen Anteilen enthält: Urea (zwischen 3 und 9 Gew.-%), Salicylsäure (zwischen 1 und 9 Ges.-%), Betamethasondipropionat (zwischen 0,1 und 0,9 Gew.-%), Propylenglycol (zwischen 1 und 9 Gew.-%) Span-80 (zwischen 3 und 12 Gew.-%), weiße Vaseline (zwischen 6 und 19 Gew.-%) Isopropylmyristat (zwischen 2 and 10 Gew.-%), Moschusrosenöl (ca. 2 bis 3 Tropfen) und Wasser.

2. Pharmazeutische Zusammensetzung für die topische Anwendung zur symptomatischen Behandlung der Psoriasis nach Anspruch 1, **dadurch gekennzeichnet, dass** sie eine Cremeform hat.

## Revendications

1. Composition pharmaceutique à usage topique pour le traitement symptomatique du psoriasis, **caractérisée en ce qu'**elle contient les ingrédients suivants dans les proportions spécifiées : urée (entre 3 et 9% M/M), acide salicylique (entre 1 et 9% M/M), dipropionate de bétaméthasone (entre 0,1 et 0,9% M/M), propylène glycol (entre 1 et 9% M/M) Span-80 (entre 3 et 12% M/M), vaseline blanche (entre 6 et 19% M/M) myristate d'isopropyle (entre 2 et 10% M/M), huile de rose musquée (environ 2 à 3 gouttes) et de l'eau.

2. Composition pharmaceutique à usage topique pour le traitement du psoriasis selon la revendication 1, **caractérisée en ce qu'**elle se présente sous la forme de crème.
